(19) Europäisches Patentamt
European Patent Office
Office européen des brevets

(11) **EP 1 337 221 B1**

(12) **EUROPÄISCHE PATENTSCHRIFT**

(45) Veröffentlichungstag und Bekanntmachung des Hinweises auf die Patenterteilung:
**08.08.2007 Patentblatt 2007/32**

(51) Int Cl.:
*A61K 6/083* (2006.01)    *A61K 6/02* (2006.01)

(21) Anmeldenummer: **01997182.9**

(86) Internationale Anmeldenummer:
**PCT/EP2001/013363**

(22) Anmeldetag: **19.11.2001**

(87) Internationale Veröffentlichungsnummer:
**WO 2002/041846 (30.05.2002 Gazette 2002/22)**

(54) **VERWENDUNG VON VERZWEIGTEN POLYSÄUREN IN DENTALMASSEN**

USE OF BRANCHED POLYACIDS IN DENTAL COMPOUNDS

UTILISATION DE POLYACIDES RAMIFIES DANS DES PRODUITS DENTAIRES

(84) Benannte Vertragsstaaten:
**AT BE CH CY DE DK ES FI FR GB GR IE IT LI LU MC NL PT SE TR**

(30) Priorität: **27.11.2000 DE 10058830**

(43) Veröffentlichungstag der Anmeldung:
**27.08.2003 Patentblatt 2003/35**

(73) Patentinhaber: **3M ESPE AG**
**82229 Seefeld (DE)**

(72) Erfinder:
• **MIKULLA, Markus**
**82346 Andechs-Frieding (DE)**
• **LECHNER, Günther**
**82237 Wörthsee (DE)**
• **STEFAN, Klaus-Peter**
**82229 Seefeld (DE)**
• **RACKELMANN, Gabriele**
**82205 Gilching (DE)**
• **ECKHARDT, Gunther**
**06231 Bad Dürrenberg (DE)**

(74) Vertreter: **Brem, Roland**
**3M ESPE AG,**
**ESPE Platz**
**82229 Seefeld (DE)**

(56) Entgegenhaltungen:
EP-A- 0 219 058        DE-A- 19 525 033
US-A- 4 174 334        US-A- 5 141 560

• DATABASE WPI Section Ch, Week 199238 Derwent Publications Ltd., London, GB; Class A14, AN 1992-313645 XP002201920 & JP 04 221304 A (NIPPON OILS & FATS CO LTD), 11. August 1992 (1992-08-11) in der Anmeldung erwähnt
• DATABASE WPI Section Ch, Week 199238 Derwent Publications Ltd., London, GB; Class A14, AN 1992-313644 XP002201921 & JP 04 221303 A (NIPPON OILS & FATS CO LTD), 11. August 1992 (1992-08-11) in der Anmeldung erwähnt

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft die Verwendung von verzweigten, nicht vernetzten, wasserlöslichen Polysäuren in Dentalprodukten, insbesondere in dentalen Zementen wie Zink-Polycarboxylatzementen, Glasionomerzementen (GIZe), Resin-Modified-Glasionomerzementen (RM-GIZe) und Compomeren, soweit sie mit wässrigen Polysäurelösungen formuliert werden können.

Polysäuren werden seit langem zur Formulierung von härtbaren Dentalmassen verwendet. Beispielsweise wurde Polyacrylsäure als Grundsubstanz in Zink-Polycarboxylatzementen (D. C. Smith, Br. Dent. J. 125 (1968), 381-384) oder auch in Glasionomerzementen (ASPA I, Wilson und Kent, 1969; DE 20 615 13 A1) eingesetzt.

Während Zink-Polycarboxytatzemente bis heute auf Polyacrylsäure basieren, wurden die Glasionomerzemente hinsichtlich der chemischen Zusammensetzung der Polysäure weiterentwickelt.

Aus S. Crisp, B. E. Kent, B. G. Lewis, A. J. Ferner und A. D. Wilson, J. Dent. Res. 59, (1980), 1055-1063 sind beispielsweise. Copolymersäuren aus Acrylsäure und Itaconsäure zur Verwendung in Glasionomerzementen bekannt. Außerdem werden in der EP 0 024.056 A1 Glasionomerzemente auf Basis eines Copolymeren aus Acrylsäure und Maleinsäure beschrieben.

Üblicherweise werden in Dentalmassen, beispielsweise bei Polyelektrolyzementen und insbesondere bei Zink-Polycarboxylatzementen und Glasionomerzementen hochkonzentrierte, beispielsweise ca. 30 bis 50 %-ige wässrige Lösungen unverzweigter Polysäuren eingesetzt.

In diesem Zusammenhang beschreibt die US 4,174,334 eine Zementzusammensetzung, umfassend gepulvertes Aluminiumboratglas und ein wasserlösliches organisches Polymer mit einer Polymerkette, die Carboxylatgruppen aufweist.

In der US 5,141,560 wird ein Dentalzement offenbart, der a) Calcium- oder Zinkoxid oder -hydroxid, b) eine substituierte aromatische Verbindung in einer bestimmten Menge, die mit der Komponente a) einen Zement bilden kann und c) ein getrocknetes Polysäurederivat enthält.

Die EP 0219 058 betrifft eine polymerisierbare Zementmischung enthaltend a) polymerisierbare ungesättigte Monomere, b) reaktive Füllstoffe sowie c) Härtungsmittel.

Hochverzweigte, teilweise vernetzte Polysäuren für den Einsatz in Dentalprodukten werden in JP 4-221303 und JP 4-221304 beschrieben. Diese Polysäuren sind jedoch nicht mehr ausreichend wasserlöslich, um für die Abbindereaktion in Dentalprodukten vollständig zur Verfügung zu stehen, so dass unreagierte Fehlstellen entstehen, die das abgebundene Material schwächen.

Der Gesamtanteil an Polysäure in dentalen Zementen muss allgemein möglichst hoch gewählt werden, um die maximalen Festigkeiten des ausgehärteten Zementes zu erreichen. Dabei muss darauf geachtet werden, dass das System beim Mischen und Applizieren eine handhabbare Viskosität aufweist. Dies ist insbesondere bei Präparaten, die in Mischkapseln vertrieben werden, wichtig, da das angemischte Material durch einen Auslassrüssel fließen muss.

Folgende Ansätze zur Optimierung sind dabei bekannt:

1. Bei GIZen wird ein Anteil der eingesetzten Polysäure ins Pulver gemischt. Der Anteil im Pulver ist aber insbesondere bei hochfesten Füllungs-GIZen begrenzt, da in der im Praxisgebrauch notwendigen kurzen Verarbeitungszeit nur wenig Polysäure aus dem Pulver beim Mischen mit der GIZ-Flüssigkeit gelöst werden kann. Nicht gelöste Anteile an Polysäure aus dem Pulver wirken im Zement als Fehlstellen und führen deshalb zu einer Schwächung des Materials.

2. Eine Verringerung des Gesamtgehaltes an Polysäure in dentalen Zementen geht allgemein und besonders bei hochfesten GIZen mit einer deutlichen Verschlechterung der mechanischen Festigkeit der ausgehärteten Zemente einher.

Die am häufigsten in Dentalprodukten eingesetzte Polysäure ist unverzweigte Polyacrylsäure. In konzentrierter wässriger Lösung hat diese Polysäure aber die Neigung zu einer irreversiblen Gelierung. Solche gelierten Polyacrylsäurelösungen sind für den Einsatz in Dentalzementen unbrauchbar. Zur Verhinderung dieses Effektes wurde den Polyacrylsäurelösungen Methanol zugesetzt, das aber einerseits giftig ist und andererseits zu Verfärbungen des Zementes im Mundmillieu führt.

3. Ein weiterer Lösungsansatz bestand darin, Copolymere aus Acrylsäure und Itaconsäure bzw. Maleinsäure für dentale Zementflüssigkeiten einzusetzen. Die Gelierung konnte damit verhindert werden. Es ergaben sich aber für die daraus hergestellten Dentalzemente einige unerwünschte Eigenschaften, wie mangelnde Eigenhaftung der Zemente an der Zahnhartsubstanz und insbesondere bei hochfesten Glasionomerzementen in der Füllungstherapie um 10 bis 20 % geringere Biegefestigkeiten und damit geringere Dauergebrauchseigenschaften.

Es besteht daher für den Einsatz in Dentalprodukten Bedarf an mit wässrigen Lösungen mischbaren Polysäuren, die keine Neigung zum Gelieren zeigen und zu hervorragenden physikalischen Eigenschaften der ausgehärteten Dentalprodukte führen.

Überraschend wurde nun gefunden, dass Polysäuren, die zu mindestens 80 Mol-% der Wiederholungseinheiten aus Acrylsäuresegmenten aufgebaut sind und gleichzeitig eine verzweigte, aber nicht vernetzte Polymerstruktur aufweisen, nicht gelieren, die aus dem Stand der Technik bekannten Nachteile vermeiden und sich daher hervorragend für den Einsatz in Dentalprodukten eignen.

Die Begriffe "umfassend" und "enthaltend" leiten im Rahmen dieser Anmeldung eine nicht-abschließende Aufzählung ein. Der Begriff "ein" ist äquivalent mit der Angabe von "mindestens ein".

Bei den Dentalmassen handelt es sich regelmäßig um härtbare Massen, d.h. um Massen, die bestimmungsgemäß in einem Zeitraum von 30 sec bis 30 min, vorzugsweise von 2 min bis 10 min von einem viskosen in einen nicht-viskosen Zustand übergehen. Die Härtungsreaktion kann beispielsweise durch eine Zementreaktion, eine Vernetzungsreaktion und/oder eine Polymerisationsreaktion bewirkt werden.

Ein viskoser, im Unterschied zu einem nicht-viskosen Zustand im obigen Sinne liegt dann vor, wenn die Masse mit dem Zahnarzt geläufigen Applikationsvorrichtungen, wie Spatel, Spritze oder Mischkapsel verarbeitet bzw. appliziert werden kann.

Unter Gelieren soll im Sinne dieser Erfindung ein Vorgang verstanden werden, bei dem die wässrige Lösung einer Polysäure aus einer fließfähigen in eine standfeste Form übergeht, wobei dieser Prozess irreversibel ist.

Unter Polysäuren sollen im Rahmen dieser Erfindung Polymere und Copolymere verstanden werden, die mehr als drei Säuregruppen pro Polymermolekül und gegebenenfalls zusätzliche andere funktionelle Gruppen aufweisen.

Unter Säuregruppen werden insbesondere Carbonsäuregruppen, Phosphonsäuregruppen, Phosphorsäuregruppen oder Sulfonsäuregruppen verstanden.

Unter verzweigten Polysäuren werden Polysäuren verstanden, die mindestens einen Verzweigungspunkt in der Polymerstruktur enthalten. Beispiele für verzweigte Polymerstrukturen sind Sternpolymere, Kammpolymere, Pfropfpolymere, langkettenverzweigte Polymere, kurzkettenverzweigte Polymere, hyperverzweigte Polymere oder Dendrimere.

Bei einer Seitengruppe, die von einer Polymerkette absteht, handelt es sich erfindungsgemäß nicht um einen Verzweigungspunkt. Auch bei durch Komplexierungsrekationen ausgelösten Vernetzungen handelt es sich nicht um Verzweigungen im Sinne der Erfindung. Von einem Verzeigungspunkt schließen sich immer Wiederholungseinheiten an. Erfindungsgemäß ist unter Verzweigungspunkt zu verstehen, eine Stelle im Polymerrückgrat, von der kovalent angebunden mindestens zwei, vorzugsweise drei Polymeräste ausgehen, wobei diese mindestens drei Wiederholungseinheiten enthalten.

Wasserlöslich im Sinne der Erfindung ist eine Polysäure dann, wenn eine wäßrige, vollständig klare Lösung, enthaltend mindestens ein Gewichtsprozent einer Polysäure, herstellbar ist. Im Gegensatz dazu sind vernetzte Polymere bekanntermaßen allgemein bei Normalbedingungen unlöslich in den im Dentalbereich gängigen Solventien.

Bevorzugt für die Erfindung sind Sternpolymere oder Kammpolymere, besonders bevorzugt Sternpolymere.

Bei Sternpolymeren sind mindestens drei für sich unverzweigte Polymerketten, vorzugsweise mit mindestens drei Wiederholungseinheiten, über einen gemeinsamen Verzweigungspunkt miteinander verknüpft. Ein Verzweigungspunkt kann ein mehrfach valentes Atom sein oder eine niedermolekulare Molekülstruktur aufweisen, wie ein Benzolring oder ein Cyclohexanring.

Bei Kammpolymeren sind mindestens zwei Polymerketten lateral an eine gemeinsame Polymerhauptkette, die für sich unverzweigt ist, geknüpft.

Erfindungsgemäß sind daher Dentalprodukte, umfassend mindestens eine Polysäure, gegebenenfalls zusammen mit mindestens einem Salz mindestens einer Polysäure, wobei die verwendeten Polysäuren mindestens einen, bevorzugt einen Verzweigungspunkt in der Polymerstruktur besitzen sowie wasserlöslich und unvernetzt sind und bezüglich der Wiederholungseinheiten zu mindestens 80 Mol-%, bevorzugt mindestens 90 Mol-%, besonders bevorzugt mindestens 95 Mol-% und ganz besonders bevorzugt 100 Mol-% aus Acrylsäuresegmenten bestehen, wobei unter Verzweigungspunkt eine Stelle im Polymerrückgrat zu verstehen ist, von der kovalent angebunden mindestens zwei Polymeräste ausgehen, wobei diese mindestens drei Wiederholungseinheiten enthalten.

Unter Wiederholungseinheiten sind nicht die Struktureinheiten der Verzweigungspunkte zu verstehen, sondern ausschließlich die sich wiederholenden Segmente der von den Verzweigungspunkten ausgehenden, vorzugsweise linearen Polymerketten. Bei Copolymersäuren sind die unterschiedlichen Wiederholungseinheiten üblicherweise statistisch angeordnet.

Die Molekulargewichtsverteilung Mw/Mn der Polysäuren liegt je nach Herstellungsmethode üblicherweise zwischen 1,0 und 10,0, vorzugsweise im Bereich von 1,5 bis 6,0; es können aber auch höhere Werte angenommen werden.

Unter Mw im Sinne dieser Anmeldung ist das gewichtsmittlere Molekulargewicht, bestimmt mittels wässriger Gelpermeationschromatographie (GPC) zu verstehen, wobei gilt:

$$Mw = \frac{\sum_i n_i M_i^{\,2}}{\sum_i n_i M_i}$$

worin $n_i$ = Zahl der Polymerketten und $M_i$ = Molmasse der Polymerkette bedeuten.

Unter Mn im Sinne dieser Anmeldung ist das zahlenmittlere Molekulargewicht, bestimmt mittels wässriger GPC zu verstehen, wobei gilt:

$$Mn = \frac{\sum_i n_i M_i}{\sum_i n_i} = \frac{\sum_i n_i M_i}{n}$$

worin n = Gesamtzahl der Polymerketten in einer Probe bedeutet.

Vorteilhaft an der erfindungsgemäßen Verwendung von verzweigten Polysäuren sind ferner die erzielbaren hohen Biegefestigkeiten. Bei Füllungs-GIZen beispielsweise sind Biegefestigkeiten erzielbar, die sich auf dem hohen Niveau der bekannten Systeme, die auf linearer Polyacrylsäure basieren, bewegen.

Mit den beschriebenen Polysäuren lassen sich härtbare Dentalmassen bzw. Zemente herstellen, die beispielsweise über eine Druck-Festigkeit im Bereich von 200 bis 260 MPa (gemessen nach ISO 9971), eine Biege-Festigkeit im Bereich von 30 bis 50 MPa (gemessen analog ISO 4049 mit 12mm langen Prüfkörpern) und/oder eine Oberflächenhärte im Bereich von 350 bis 510 MPa (gemessen nach DIN 53456) aufweisen.

Ein weiterer Vorteil der erfindungsgemäßen Dentalprodukte enthaltend verzweigte Polysäuren liegt darin, dass in konzentrierter wässriger Lösung der verzweigten Polysäuren deutlich verminderte Viskositäten gefunden werden, was zu einer vereinfachten Anmischbarkeit von Pulver und Flüssigkeit führt.

Darüber hinaus bieten die erfindungsgemäßen Dentalprodukte den Vorteil, dass keine toxischen oder andere unerwünschten Bestandteile oder Zusätze enthalten sind.

Die in den erfindungsgemäßen Dentalprodukten enthaltenen verzweigten Polysäuren sind grundsätzlich nach verschiedenen Herstellungsmethoden zugänglich.

Für Sternpolymere bekannte Syntheseprinzipien "Arm-first" und "Core-first" (K. Matyjaszewski et al, Macromolecules 1999, 32, S. 6526) gelten prinzipiell auch für Kammpolymere.

1. Stern- und Kammpolymersäuren können nach der "Arm-first-Methode"- aus linearen Polysäuren oder deren in Polysäuren überführbaren Derivaten, die ein funktionelles Kettenende besitzen, durch reaktive Anknüpfung an eine mehrfach-funktionelle niedermolekulare, beispielsweise cyclische Verbindung oder oligomere, beispielsweise macrocyclische oder linear polymere Verbindung hergestellt werden. Als allgemeine Beispiele seien genannt:

a) Herstellung von Polyacrylsäure mit Aminoendgruppe, beispielsweise durch radikalische Polymerisation in Gegenwart von aminofunktionellem Übertragungsreagenz (A. Akar, N. Öz, Angew. Makromol. Chem. 273, 1999, 12-14) und anschließende Umsetzung beispielsweise mit mehrfach funktionellen Carbonsäurechloriden oder -anhydriden, wie Benzoltricarbonsäure-trichlorid, Butan-tetracarbonsäure-tetrachlorid, oligomerem Maleinsäureanhydrid, Acrylsäurechloridpolymeren, wie Poly-(acrylsäure-co-acrylsäurechlorid), mehrfach funktionellen Benzylhalogeniden, wie 1,2,4,5-Tetrakis-brommethylbenzol, Hexakisbrom-methylbenzol, mit Tetrabrommethyl-pentaerythrit oder anderen niedermolekularen, oligomeren oder polymeren Verknüpfungsreagenzien. Zur Durchführung der Verknüpfungsreaktion eignet sich häufig 1,4-Dioxan besonders gut, da es ein gutes Lösungsmittel für Polyacrylsäure darstellt.

b) Als funktionelle Polysäurederivate können auch Polyacryläurederivate, wie Polyacrylsäure-tert.-butylester mit Endgruppenfunktionalisierung verwendet werden, wobei als Endgruppen neben den bevorzugten primären oder sekundären Aminogruppen auch beispielsweise Alkohol- oder Thiolgruppen in Frage kommen. Nach einer Verknüpfung dieser Polymere analog 1.a) müssen die tert.-Butylestergruppen selektiv gespalten werden. Dies kann beispielsweise durch Erhitzen des Polymers in Dioxanlösung mit Salzsäure geschehen (K. A. Davis et al, Polymer Prepr. 2/1999, 430).

c) Als Polyacrylsäurederivat kann auch durch anionische Polymerisation hergestelltes Poly-(butylacrylat) (P. Banerjee und A. M. Mayes, Macromolecules 1998, 31, 7966) verwendet werden, das als anionische Spezies nach 1.a) mit einem Verknüpfungsreagenz umgesetzt wird. Anschließend werden nach üblichen Methoden die Butylestergruppen abgespalten, beispielsweise durch saure oder alkalische Hydrolyse in einem wässrig-organischen Medium, wie einem Dioxan-WasserGemisch.

2. Stern- und Kammpolymersäuren können nach der "Core-first-Methode" durch Aufpolymerisieren von Säuremonomeren oder deren in Säuresegmente überführbare Derivate auf mehrfach funktionelle niedermolekulare, cyclische, oligomere, macrocyclische oder linear polymere Startersubstanzen nach einem lebenden Polymerisationsmechanismus, bevorzugt anionisch oder lebend radikalisch, hergestellt werden. Der lebende Polymerisationsmechanismus vermeidet hierbei die Bildung höher verzweigter oder vernetzter Polymere. Bei der lebenden radikalischen Polymerisation kann bevorzugt die Atom-Transfer-Polymerisation (ATRP) zur Anwendung kommen.

a) Mehrfach funktionelle Startersubstanzen zur Herstellung von Acrylatsternpolymeren werden beispielsweise in C. Pugh et al, Polymer Prepr. 2/1999, 108; D. M. Haddleton et al, New J. Chem. 1999, 23, 477; Heise et al, Polymer Prepr. 1/1999, 452; A. Heise et al, Macromolecules 1999, 32, 231; S. Angot et al, Macromolecules 1998, 31, 7218 beschrieben. Die anschließend notwendige Spaltung der Polyacrylatestergruppen kann nach üblichen Methoden erfolgen.

b) Mehrfach funktionelle Startersubstanzen für die Synthese von Acrylatkammpolymeren können beispielsweise Copolymere oder Oligomere aus Acrylaten und 4-Chlormethylstyrol sein, wobei die benzylischen Chloridgruppen als ATRP-Starter für Acrylate geeignet sind. Nach der ATRP können die Acrylatgruppen in der Hauptkette und in den Nebenketten zu Acrylsäuresegmenten nach üblichen Methoden gespalten werden.

Zusätzlich zu den Acrylsäuresegmenten können weitere saure Wiederholungseinheiten oder auch andere funktionelle oder nicht funktionelle Wiederholungseinheiten und Gruppen in den Polysäuren enthalten sein, in der Summe von 0,1 bis 20 Mol-%, bevorzugt 0,1 bis 10 Mol-%, besonders bevorzugt 0,1 bis 5 Mol-%.
Die weiteren sauren Wiederholungseinheiten können beispielsweise hervorgegangen sein aus der Copolymerisation von Acrylsäure mit Methacrylsäure, Maleinsäure, Maleinsäureanhydrid, Fumarsäure, Itaconsäure, Itaconsäureanhydrid, Glutaconsäure, Aconitsäure, Citraconsäure, Methaconsäure, Tiglinsäure, Crotonsäure, Muconsäure, Isocrotonsäure, 3-Butensäure, Zimtsäure, Styrolcarbonsäure, Vinylphthalsäure, Abietinsäure, Styrolsulfonsäure, Styrolphosphonsäure, 1-Phenylvinylphosphonsäure, Vinylphosphonsäure, Vinylidendiphosphonsäure, Vinylsulfonsäure, Phosphorsäurevinylester oder anderen säurefunktionellen Monomeren sowie deren substituierten Derivaten, insbesondere Estern oder Amiden bzw. Imiden der genannten Säuren mit bis zu 10 C-Atomen im Alkoholatrest bzw. Aminrest oder Iminrest, beispielsweise Maleinimid, N-Methylmaleinimid, N-Ethylmaleinimid, Acrylsäureamid, N-Methyl-acrylsäureamid, N,N-Dimethylacrylasäureamid, N-Ethylacrylsäureamid, Methylacrylat, Ethylacrylat, n-Propylacrylat, i-Propylacrylat, n-Butylacrylat, i-Butylacrylat, tert.-Butylacrylat.
Beispiele für andere funktionelle Wiederholungseinheiten sind Struktursegmente hervorgegangen aus Vinylacetat, Vinylethern, beispielsweise Vinyl-methylether, Vinylalkohol, Butadien, Isopren, Styrol, Vinylchlorid, Vinylidendichlorid, Vinylfluorid, Vinylidendifluorid.
Beispiele für nicht funktionelle Wiederholungseinheiten sind Struktureinheiten hervorgegangen aus Ethylen, Propylen, Tetramethylen, 1-Buten, 1-Penten, 1-Hexen.
Die Polysäuren können je nach Anwendungsgebiet als Flüssigkeit oder gegebenenfalls zusätzlich als Feststoff, beispielsweise erhalten durch Gefrier- oder Sprühtrocknung, eingesetzt werden.
Die Polysäuren weisen üblicherweise ein Molekulargewicht Mw im Bereich von 1000 bis 1.000.000, vorzugsweise im Bereich von 10.000 bis 500.000 auf.
Bevorzugt werden die Polysäuren als wässrige Lösungen mit 1 bis 70 Gew.-%, bevorzugt 20 bis 60 Gew.-%, besonders bevorzugt 30 bis 50 Gew.-% eingesetzt, bei einer Temperatur von 22°C.
Die Kationen der Salze der Polysäuren, die gemäß der Erfindung eingesetzt werden können, sind entnommen aus der Gruppe: Alkali- und Erdalkalielemente, Zink, Aluminium, Scandium, Yttrium, Lanthan. Bevorzugt sind hierbei Na-, Ca- und Al-Salze.
Bevorzugte Dentalmassen im Rahmen dieser Erfindung sind entweder einkomponentig formuliert, wie Compomere, oder zwei- oder mehrkomponentig formuliert, wie Carboxylatzemente, Glasionomerzemente und Resin-Modified-Glasionomerzemente, bei denen die flüssigen von den festen Komponenten getrennt gelagert und direkt vor der Applikation gemischt werden.
Durch eine verringerte Lösungsviskosität der erfindungsgemäßen Polysäuren sind bei gleicher Polysäure-Konzentration höhere P/F-Verhältnisse einstellbar und noch gut handhabbar (bis etwa 30% höher) als bei den aus dem Stand der Technik bekannten Polysäuren. Dies führt regelmäßig auch zu verbesserten Materialfestigkeiten (um bis etwa 20 % verbesserte Werte bei der Druck- und Biegefestigkeit).

Brauchbare Pulver/Flüssigkeitsverhältnisse liegen im Bereich von 0,7 bis 6, vorzugsweise im Bereich von 1 bis 5, besonders bevorzugt im Bereich von 1,5 bis 3,5.

Besonders bevorzugt sind Zemente, die im Falle von Glasionomerzementen beispielsweise folgende Bestandteile umfassen können:

(A) 1 bis 60, bevorzugt 5 bis 40 Gew.-%, besonders bevorzugt 10 bis 30 Gew.-% Polysäuren, gegebenenfalls zusammen mit mindestens einem Salz mindestens einer Polysäure, wobei die verwendeten Polysäuren mindestens einen, bevorzugt einen Verzweigungspunkt in der Polymerstruktur besitzen sowie wasserlöslich und unvernetzt sind und bezüglich der Wiederholungseinheiten zu mindestens 80 Mol-%, bevorzugt 90 Mol-%, besonders bevorzugt 95 Mol-% und ganz besonders bevorzugt 100 Mol-% aus Acrylsäuresegmenten bestehen;

(B) 35 bis 80, bevorzugt 50 bis 70 Gew.-% Füllstoffe;

(C) 0 bis 20, bevorzugt 1 bis 10 Gew.-% Zusatz- und Hilfsstoffe;

(D) 5 bis 40, bevorzugt 9 bis 30 Gew.-% Wasser.

Unter Füllstoffen der Komponente (B) sind prinzipiell reaktive oder nicht reaktive Feststoffe zu verstehen,

Geeignet sind beispielsweise reaktive Fluoraluminiumsilkatgläser aus DE 20 61 513 A, DE 20 65 824 A oder reaktive Gläser, die an der Oberfläche im Vergleich zur durchschnittlichen Zusammensetzung an Calciumionen verarmt sind, wie in DE 29 29 121 A beschrieben.

Letzt genannte Gläser sind besonders bevorzugt und können folgende Zusammensetzung aufweisen:

| Bestandteil | Berechnet als | Gew.-% |
|---|---|---|
| Si | $SiO_2$ | 20 bis 60 |
| Al | $Al_2O_3$ | 10 bis 50 |
| Ca | CaO | 1 bis 40 |
| F | F | 1 bis 40 |
| Na | $Na_2O$ | 0 bis 10 |
| P | $P_2O_5$ | 0 bis 10 |

sowie zusätzlich insgesamt 0 bis 20 Gew.-%; berechnet als Oxide, an B, Bi, Zn, Mg, Sn, Ti, Zr, La oder anderen dreiwertigen Lanthanoiden, K, W, Ge, sowie weiteren Zusätzen, welche die Eigenschaften nicht beeinträchtigen und physiologisch unbedenklich sind.

Zusätzlich zu den vorher beschriebenen reaktiven Gläsern können inerte Füllstoffe, wie Quarze, eingesetzt werden.

Unter Komponente (C) sind beispielsweise Zusätze zur Beschleunigung und Verbesserung der Aushärtung, wie sie aus DE 2 319 715 A bekannt sind, zu verstehen. Vorzugsweise werden chelatbildende Mittel in Form niedermolekularer Säuremoleküle, wie Weinsäure, zugesetzt.

Ebenso sind darunter Farbpigmente und andere im GIZ-Bereich übliche Hilfsstoffe, beispielsweise zur Verbesserung der Anmischbarkeit, zu verstehen. Neben der Verwendung in konventionellen Glasionomerzementen eignen sich die hier beschriebenen Polysäuren auch für den Einsatz in Carboxylatzementen.

Hierbei umfassen die Zusammensetzungen beispielsweise folgende Bestandteile:

(A) 1 bis 60, bevorzugt 5 bis 40 Gew.-% Polysäuren, besonders bevorzugt 10 bis 30 Gew.-%, gegebenenfalls zusammen mit mindestens einem Salz mindestens einer Polysäure, wobei die verwendeten Polysäuren mindestens einen, bevorzugt einen Verzweigungspunkt in der Polymerstruktur besitzen sowie wasserlöslich und unvernetzt sind und bezüglich der Wiederholungseinheiten zu mindestens 80 Mol-%, bevorzugt 90 Mol-%, besonders bevorzugt 95 Mol-% und ganz besonders bevorzugt 100 Mol-% aus Acrylsäuresegmenten bestehen;

(C) 0 bis 20, bevorzugt 1 bis 10 Gew.-% Zusatz- und Hilfsstoffe;

(D) 10 bis 40, bevorzugt 15 bis 30 Gew.-% Wasser;

(E) 30 bis 80, bevorzugt 44 bis 70 Gew.-% Zinkoxid.

Compomere oder Resin-Modified-GIZe, die die hier beschriebenen Polysäuren enthalten, umfassen beispielsweise folgende Komponenten:

(A) 1 bis 76 bevorzugt 2 bis 69,9 Gew.-%, besonders bevorzugt 10 bis 40 Gew.-%, Polysäuren, gegebenenfalls zusammen mit mindestens einem Salz mindestens einer Polysäure, wobei die verwendeten Polysäuren mindestens einen, bevorzugt einen Verzweigungspunkt in der Polymerstruktur besitzen sowie wasserlöslich und unvernetzt sind und bezüglich der Wiederholungseinheiten zu mindestens 80 Mol-%, bevorzugt 90 Mol-%, besonders bevorzugt 95 Mol-% und ganz besonders bevorzugt 100 Mol-% aus Acrylsäuresegmenten bestehen;

(D) 5 bis 40, bevorzugt 5 bis 35 Gew.-% Wasser;

(F) 8,9 bis 70, bevorzugt 10 bis 60 Gew.-% eines oder mehrerer radikalisch polymerisierbarer Monomere;

(G) 10 bis 90, bevorzugt 15 bis 87,9 Gew.-% Füllstoffe;

(H) 0,1 bis 5, bevorzugt 0,5 bis 3 Gew.-% Initiatoren und gegebenenfalls Aktivatoren;

(I) 0 bis 30, bevorzugt 0,1 bis 20 Gew.-% Additive, gegebenenfalls Pigmente, Thixotropiehilfsmittel, Weichmacher.

Als Komponente (F) werden mono-, di-, oder höherfunktionelle ethylenisch ungesättigte Verbindungen, bevorzugt auf Acrylat- und/oder Methacrylatbasis eingesetzt. Diese können sowohl monomere als auch höhermolekulare oligomere oder polymere Acrylate beinhalten. Ferner können sie in alleiniger Form oder in Abmischung in den Formulierungen eingesetzt werden.

Geeignete Monomere sind beispielsweise die Acrylsäure- und Methacrylsäureester mono-, di- oder höherfunktioneller Alkohole. Exemplarisch seien genannt: 2-Hydroxyethyl(meth)acrylat, Methyl(meth)acrylat, iso-Butyl(meth)acrylat, Ethylenglykoldi(meth)acrylat, Triethylenglykoldi(meth)acrylat (TEGDMA), Hexandioldi(meth)-acrylat, Dodecandioldi(meth)acrylat und Trimethylolpropantri(meth)acrylat.

Vorteilhaft einsetzbar sind weiterhin Bisphenol-A-di(meth)acrylat sowie die davon abgeleiteten ethoxy- bzw. propoxylierten Di(meth)acrylate. Auch die in der US 3 066 112 beschriebenen Monomere auf Basis von Bisphenol-A- und Glycidyl-(meth)acrylat oder deren durch Addition von Isocyanaten entstandenen Derivate sind geeignet.

Besonders geeignet sind auch die in der DE 28 16 823 C genannten Diacryl- und Dimethacrylsäureester des Bis(hydroxymethyl)-tricyclo$[5.2.1.0^{2,6}]$-decans und die Diacryl- und Dimethacrylsäureester der mit 1 bis 3 Ethylenoxid- und/oder Propylenoxideinheiten verlängerten Verbindungen des Bis(hydroxymethyl)-tricyclo$[5.2.1.0^{2,6}]$-decans.

Auch Urethan(meth)acrylate, wie 7,7,9-Trimethyl-4,13-dioxo-5,12-diazahexadecan-1,16-dioxy-dimethacrylat (UDMA), können Bestandteil dieser Komponente sein.

Füllstoffe gemäß Komponente (G) können anorganische Füllstoffe, beispielsweise Quarz, gemahlene Gläser, nicht wasserlösliche Fluoride, wie $CaF_2$, Kieselgele sowie Kieselsäure, insbesondere pyrogene Kieselsäure und deren Granulate sein. Auch Christobalit, Calciumsilikat, Zirkoniumsilikat, Zeolithe, einschließlich der Molekularsiebe, Metalloxidpulver, wie Aluminium- oder Zinkoxide bzw. deren Mischoxide, Bariumsulfat, Yttriumfluorid, Calciumcarbonat sind als Füllstoffe einsetzbar.

Auch fluoridauslösende Füllstoffe, beispielsweise komplexe anorganische Fluoride der allgemeinen Formel $A_nMF_m$ wie in der DE 44 45 266 A beschrieben, können eingesetzt bzw. zugesetzt werden. Dabei bedeutet A ein ein- oder mehrwertiges Kation, M ein Metall der III, IV, V Haupt- oder Nebengruppe, n eine ganze Zahl von 1 bis 3 und m eine ganze Zahl von 4 bis 6.

Bestandteil dieser Komponente können auch organische Füllstoffe sein. Exemplarisch genannt seien übliche perlförmige Polymere und Copolymere auf Basis von Methylmethacrylat, die beispielsweise bei der Fa. Röhm unter der Bezeichnung "Plexidon" oder "Plex" erhältlich sind.

Zum besseren Einbau in die Polymermatrix kann es von Vorteil sein, die genannten Füllstoffe sowie gegebenenfalls röntgenopake Zusatzstoffe mittels eines Silans zu hydrophobieren. Die Menge des eingesetzten Silans beträgt üblicherweise 0,5 bis 10 Gew.-%, bezogen auf anorganische Füllstoffe, bevorzugt 1 bis 6 Gew.-%, ganz besonders bevorzugt 2 bis 5 Gew.-%, bezogen auf anorganische Füllstoffe. Übliche Hydrophobierungsmittel sind Silane, beispielsweise Trimethoxymethacryloxypropylsilan. Die maximale mittlere Korngröße der anorganischen Füllstoffe beträgt vorzugsweise 15 μm, insbesondere 8 μm. Ganz besonders bevorzugt werden Füllstoffe mit einer mittleren Korngröße von < 3 μm eingesetzt.

Unter Initiatoren gemäß Komponente (H) sind Initiatorsysteme, die die radikalische Polymerisation der Monomeren bewirken, beispielsweise Photoinitiatoren und/oder sogenannte Redoxinitiatorsysteme und/oder thermische Initiatoren zu verstehen.

Als Photoinitiatoren eignen sich beispielsweise α-Diketone, wie Campherchinon, in Verbindung mit sekundären und tertiären Aminen, oder Mono- und Bisacylphosphinoxide, wie 2,4,6-Trimethylbenzoyldiphenyl-phosphinoxid und Bis-(2,6-dichlorbenzoyl)-4-n-propylphenyl-phosphinoxid. Es eignen sich aber auch andere Verbindungen dieses Typs, wie sie in EP 0 073 413 A, EP 0 007 508 A, EP 0 047 902 A, EP 0 057 474 A und EP 0 184 095 A beschrieben sind.

Als Redoxinitiatorsysteme eignen sich organische Peroxidverbindungen zusammen mit sogenannten Aktivatoren. Als organische Peroxidverbindungen kommen dabei insbesondere Verbindungen wie Lauroylperoxid, Benzoylperoxid sowie p-Chlorbenzoylperoxid und p-Methylbenzoylperoxid in Betracht.

Als Aktivatoren eignen sich beispielsweise tertiäre aromatische Amine, wie die aus der US 3 541 068 bekannten N,N-Bis-(hydroxyalkyl)-3,5-xylidine sowie die aus der DE 26 58 530 A bekannten N,N-Bis-(hydroxyalkyl)-3,5-di-t-butylaniline, insbesondere N,N-Bis-(β-oxybutyl)-3,5-di-t-butylanilin sowie N,N-Bis-(hydroxyalkyl)-3,4,5-trimethylaniline.

Gut geeignete Aktivatoren sind auch die in der DE 14 95 520 C beschriebenen Barbitursäuren und Barbitursäurederivate sowie die in der EP 0 059 451 A beschriebenen Malonylsulfamide. Bevorzugte Malonylsulfamide sind 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Dioctyl-4-isobutylmalonylsulfamid.

Zur weiteren Beschleunigung wird die Polymerisation hierbei vorzugsweise in Gegenwart von Schwermetallverbindungen und ionogenem Halogen oder Pseudohalogen durchgeführt. Als Schwermetall ist Kupfer, als Halogenid das Chloridion besonders geeignet. Das Schwermetall wird geeigneterweise in Form löslicher organischer Verbindungen eingesetzt. Ebenso werden die Halogenid- und Pseudohalogenidionen geeigneterweise in Form von löslichen Salzen eingesetzt. Beispielsweise genannt seien die löslichen Aminhydrochloride sowie quartäre Ammoniumchloridverbindungen.

Wenn die erfindungsgemäßen Dentalmassen ein Redoxinitiatorsystem aus organischem Peroxid und Aktivator enthalten, so sind vorzugsweise Peroxid und Aktivator in räumlich voneinander getrennten Teilen der erfindungsgemäßen Dentalmasse vorhanden, die erst unmittelbar vor der Anwendung homogen miteinander vermischt werden. Liegen nebeneinander organisches Peroxid, Kupferverbindung, Halogenid und Malonylsulfamid und/oder Barbitursäure vor, so ist es insbesondere sinnvoll, dass organisches Peroxid, Malonylsulfamid und/oder Barbitursäure und die Kombination Kupferverbindung/Halogenid in drei räumlich voneinander getrennten Bestandteilen vorliegen. Beispielsweise können die Kombination Kupferverbindung/Halogenid, polymerisierbare Monomere sowie Füllstoffe zu einer Paste verknetet sein und die anderen Komponenten in oben beschriebener Weise jeweils mit einer geringen Menge an Füllstoffen oder insbesondere Thixotropie-Hilfsmitteln, wie silanisierter Kieselsäure, und einem Weichmacher, beispielsweise Phthalat, zu zwei separaten Pasten verknetet sein. Andererseits können die polymerisierbaren Monomeren auch zusammen mit organischem Peroxid und Füllern vorliegen. Alternativ ist auch eine Aufteilung von organischem Peroxid, Kupferverbindung, Halogenid und Malonylsulfamid und/oder Barbitursäure gemäß DE 199 28 238 A realisierbar.

Als Vertreter der Komponente (I) können beispielsweise zur Erhöhung der Flexibilität der Massen lösliche organische Polymere eingesetzt werden. Geeignet sind beispielsweise Polyvinylacetat sowie die Copolymeren auf Basis Vinylchlorid/Vinylacetat, Vinylchlorid/Vinylisobutylether und Vinylacetat/Maleinsäuredibutylether. Gut geeignet als zusätzliche Weichmacher sind beispielsweise Dibutyl-, Dioctyl- und Dinonylphthalate oder -adipate sowie höhermolekulare Polyphthalsäure- und Adipinsäureester. Als Thixotropiehilfsmittel können neben pyrogenen Kieselsäuren auch modifizierte Schichtsilikate (Bentonite) oder organische Modifikatoren, beispielsweise auf Basis hydrierter Rizinusöle eingesetzt werden. Als Additive können weiterhin Verzögerer, wie sie in der EP 0 374 824 A als Komponente (d) beschrieben sind, in den Formulierungen enthalten sein.

Erfindungsgemäß sind ebenfalls Behältnisse umfassend mindestens eine komponente einer dentalmasse gemäss den Ansprüchen 1-9. Derartige Behältnisse können beispielsweise Kapseln, Tuben, Schraubtuben, Applikationsspritzen und - spitzen, Kanülen, Beutel, Blisterverpackungen, Becher oder Gläser sein. Prinzipiell eignen sich alle Behältnisse, in denen mehrere Komponenten getrennt voneinander gelagert werden können, wobei unter getrennter Lagerung auch die physikalische Trennung der Bestandteile über den unterschiedlichen Aggregatzustand verstanden werden soll.

Die Erfindung wird nachfolgend durch Beispiele erläutert, ohne dass sie durch diese begrenzt werden soll.

Die Bestimmung der Molmassenverteilung erfolgte hierbei über wässrige Gelpermationschromatographie (GPC) bei 23°C und pH = 7 gegenüber Polyacrylsäure-Na-Standard mit dem Brechungsindexdetektor RID6a (Fa. Shimadzu) und vergleichender Messung mit dem Lichtstreudetektor MiniDawn (Fa. Wyatt), wobei letztere Messung als Absolutmessmethode den Unterschied zwischen unverzweigten und verzweigten Polysäuren ermöglicht. Zur Eichung des Brechungsindexdetektors wurden Polyacrylsäure-Na-Standards (Fa. PSS) eingesetzt. Die Messwerte wurden mit dem Faktor 0,766 auf freie Polyacrylsäure umgerechnet.

Die Proben wurden dabei als 0,05%-ige wässrige Lösungen im Laufmittel, einer 0,9 Gew.-%-igen wässrigen Natriumnitratlösung, der noch 200 ppm Natriumazid zugesetzt sind, vermessen. Die Lösungen wurden durch Zugabe von Natriumhydroxid auf pH = 7 eingestellt.

Für die Messung von maximalen Molekulargewichten bis zu 670.000 g/mol wurde eine Säulenkombination aus Hema3000, HemaBio1000 und HemaBio40 (Fa. PSS) verwendet. Bei maximalen Molekulargewichten bis 1.100.000 g/mol wurde eine Säulenkombination aus Suprema1000, Hema3000 und HemaBio1000 (Fa. PSS) eingesetzt.

Herstellungsbeispiel 1

4-Arm-Stem-Polyacrylsäure (PAS-4)

Unter Stickstoffatmosphäre werden 5,0 g (11,1 mmol) 1,2,4,5-Tetrakis-brommethylbenzol, 18,2 g (211 mmol) Methylacrylat, 0,27 g Kupferpulver, 1,56 g Kupfer-II-trifiat und 1,46 g N,N,N',N",N"-Pentamethyl-diethylentriamin zusammengegeben. Das dunkle Gemisch wird unter Rühren 17 Stunden auf 80 °C erhitzt. Ein $^1$H-NMR-Spektrum zeigt danach vollständige Reaktion der Benzylbromidgruppen an (Verschwinden des Signals bei 4,6 ppm), so dass von einem gleichberechtigten Wachstum der vier Arme ausgegangen werden kann.

Nach Zugabe von 200 g Methylacrylat wird weitere 20 Stunden auf 80 °C erhitzt. Das Reaktionsgemisch wird dabei zähflüssig. Mittels eines $^1$H-NMR-Spektrums wird ein Monomerumsatz von ca. 98 % ermittelt.

Das Polymerisat wird in 2 Liter Methylenchlorid gelöst und nacheinander mit 2 molarer Salzsäure sowie Wasser extrahiert. Das letzte Waschwasser hat einen pH-Wert von 5.

Zur Verseifung wird die organische Lösung mit einem Volumenteil wässriger 4 molarer Kalilauge versetzt und unter heftigem Rühren auf 40 °C erhitzt. Der Umsatz wird über $^1$H-NMR-Spektroskopie verfolgt und ist nach 48 Stunden vollständig. Die wässrige Phase wird abgetrennt und mehrfach mit saurem Ionenaustauscher behandelt.

Durch Einengen im Vakuum und Kontrolle mittels Titration mit Lithiumhydroxid wird eine Konzentration von 40 Gew.-% eingestellt. Gemäß Atomabsorptionsspektroskopie liegt der Kaliumgehalt unterhalb von 100 ppm.

Ausbeute: 424 g (entspricht 92 % Polymerausbeute).

Mittels Brechungsindexdetektor gegen linearen Polyacrylsäure-Na-Standard (PAS) bzw. mit Lichtstreudetektor (LS) wurden folgende Werte ermittelt:

$$M_w = 133000 \text{ (LS)} \quad \text{bzw. } 88000 \text{ (PAS)}$$
$$M_n = 78000 \text{ (LS)} \quad \text{bzw. } 44000 \text{ (PAS)}$$
$$M_w/M_n = 1.7 \text{ (LS)} \quad \text{bzw. } 2.0 \text{ (PAS)}$$

Herstellungsbeispiel 2:

6-Arm-Stern-Polvacrylsäure (PAS-6)

Unter Stickstoffatmosphäre werden 1.0 g Hexakis-(brommethyl)-benzol mit 180 mg Kupferpulver, 1020 mg Kupfer-II-triflat, 960 mg N,N,N',N",N"-Pentamethyl-diethylentriamin und 93,0 g Methylacrylat nacheinander in einen Kolben eingewogen. Das Gemisch wird unter Rühren auf 80 °C erhitzt. Nach fünf Tagen Reaktionszeit ist der dunkle Kolbeninhalt stark zähflüssig. Mittels $^1$H-NMR-Spektroskopie wird ein Umsatz von > 90 % ermittelt.

Der Ansatz wird in 2 Liter Methylenchlorid gelöst und nacheinander mit 2 molarer Salzsäure und Wasser extrahiert, so dass das letzte Waschwasser einen pH-Wert von 5 hat.

Die organische Phase wird mit einem Volumenteil 4 molarer Kalilauge versetzt und unter heftigem Rühren 3 Tage auf 40 °C erhitzt. Der Umsatz der Verseifung ist laut $^1$H-NMR danach vollständig. Die wässrige Phase wird mit saurem Ionenaustauscher behandelt und durch Einengen im Vakuum auf eine Konzentration von 32 Gewichtsprozent gebracht (Titration mit LiOH). Der Kaliumgehalt liegt nach Atomabsorptionsspektroskopie unterhalb 100 ppm.

Ausbeute: 197 g (entspricht 81 % Polymerausbeute)

Mittels Brechungsindexdetektor gegen linearen Polyacrylsäure-Na-Standard (PAS) bzw. mit Lichtstreudetektor (LS) wurden folgende Werte ermittelt:

$$M_w = 460000 \text{ (LS)} \quad \text{bzw. } 117000 \text{ (PAS)}$$
$$M_n = 182000 \text{ (LS)} \quad \text{bzw. } 47000 \text{ (PAS)}$$
$$M_w/M_n = 2,5 \text{ (LS)} \quad \text{bzw. } 2,5 \text{ (PAS)}$$

Die GPC-Ergebnisse bestätigen die Sternstruktur.

Anwendungsbeispiele

Die Polysäuren gemäß den Herstellungsbeispielen wurden in der Anwendung für dentale Glasionomerzemente geprüft.

Biegefestigkeiten wurden in Anlehnung an die ISO-Norm 4049 bestimmt, wobei aber 12 mm lange Probekörper verwendet wurden. Es wurden jeweils Mittelwerte aus Serien von fünf Prüfkörpern bei einem relativen Fehler von ca. $\pm$ 10 % pro Messwert bestimmt. Zur Bestimmung von Druckfestigkeiten der abgebundenen Zemente wurde nach der ISO- Norm 9917 verfahren. Oberflächenhärten wurden analog nach DIN 53456 bestimmt, wobei die Prüfkörper bis zur Messung bei 36°C im Wasser gelagert wurden.

Die Viskositäten der wässrigen Polysäurelösungen wurden mit dem Viskosimeter PK 100 der Firma Haake bei 23°C gemessen.

Konzentrationen wurden durch Titration mit Lithiumhydroxid in Gegenwart von 1 Gew.-% Lithiumchlorid bestimmt.

Die Lagerstabilität wurde durch Einlagerung der Polysäurelösungen in Glasflaschen bei Raumtemperatur (25°C) und visuelle Begutachtung der Proben in regelmäßigen Zeitabständen ermittelt. Von jeder Polysäure wurden mehrere Chargen eingelagert, wobei die analytischen Werte der verschiedenen Chargen von jeweils einer Polysäure sich nur geringfügig unterschieden (Abweichungen maximal < 10 %).

Die angegebenen Polysäuren wurden mit einem Standardglasionomerpulver auf Basis eines Calcium-Strontium-Aluminium-Fluorosilikatglases-(GIZ-Glas) (Diamond-Carve-Pulver, Fa. Kemdent, England) mit einem Pulver/Flüssigkeitsverhältnis von 3,5/1 (Gewichtsteile) angespatelt.

Als Vergleichsbeispiel dienten zwei kommerzielle (Fa. Aldrich), unverzweigte Polyacrylsäuren (PAS-1a und PAS-1b) mit unterschiedlichen mittleren Molmassen.

Die Ergebnisse sind in Tabelle 1 zusammengefasst:

Tabelle 1:

| Polysäure | PAS-1a | PAS-1b | PAS-4 (erfindungsgemäß) | PAS-6 (erfindungsgemäß) |
|---|---|---|---|---|
| **Konzentration [%] ($\pm$1)** | 42 | 35 | 44 | 40 |
| **Mw (GPC-LS)** | 60000 | 250000 | 133000 | 460000 |
| **Mn (GPC-LS)** | 12000 | 50000 | 78000 | 182000 |
| **Viskosität [Pa.s]** | 8,0 | 8,2 | 8,4 | 7,7 |
| **Biegefestigkeit [MPa]** | 42 | 45 | 47 | 44 |
| **Lagerstabilität gegenüber Gelieren** | < 6 Monate (3 von 5 Chargen) | < 6 Monate (2 von 3 Chargen) | > 24 Monate (alle Chargen) | > 24 Monate (alle Chargen) |

Trotz deutlich höherer mittlerer Molmassen und höherer Konzentrationen der Stempolysäuren liegen die Biegefestigkeiten und die Viskositäten auf gleichem Niveau wie bei den Vergleichssubstanzen (Vergleich: PAS-4 mit PAS-1a und PAS-6 mit PAS-1b). Dabei zeigen die Sternsäuren jedoch eine zuverlässige Lagerstabilität über 24 Monate, während von den unverzweigten Vergleichssäuren mehrere Chargen bereits innerhalb von sechs Monaten irreversibel gelierten und damit unbrauchbar wurden.

**Patentansprüche**

1. Dentalmassen, enthaltend mindestens eine Polysäure oder mindestens eine Polysäure zusammen mit mindestens einem Salz von mindestens einer Polysäure, wobei die eingesetzten Polysäuren mindestens einen Verzweigungspunkt in der Polymerstruktur besitzen sowie wasserlöslich und unvernetzt sind und bezüglich der Wiederholungseinheiten zu mindestens 80 Mol-% aus Acrylsäuresegmenten bestehen, wobei unter Verzweigungspunkt eine Stelle im Polymerrückgrat zu verstehen ist, von der kovalent angebunden mindestens zwei Polymeräste ausgehen, wobei diese mindestens drei Wiederholungseinheiten enthalten.

2. Dentalmassen nach Anspruch 1, wobei die eingesetzten Polysäuren Sternpolymere oder Kammpolymere darstellen.

3. Dentalmassen nach einem der vorangehenden Ansprüche, wobei die Polymersäuren Copolymere darstellen, wobei der Anteil der Comonomeren 0,1 bis 20 Mol-% beträgt.

4. Dentalmassen nach Anspruch 1, umfassend:

(A) 1 bis 60 Gew.-% Polysäuren; wobei die verwendeten Polysäuren mindestens einen Verzweigungspunkt in der Polymerstruktur besitzen sowie wasserlöslich und unvernetzt sind und bezüglich der Wiederholungseinheiten zu mindestens 80 Mol-% aus Acrylsäuresegmenten bestehen, wobei unter Verzweigungspunkt eine Stelle im Polymerrückgrat zu verstehen ist, von der kovalent angebunden mindestens zwei Polymeräste ausgehen, wobei diese mindestens drei Wiederholungseinheiten enthalten;
(B) 35 bis 80 Gew.-% Füllstoffe;
(C) 0 bis 20 Gew.-% Zusatz- und Hilfsstoffe;
(D) 5 bis 40 Gew.-% Wasser.

5. Dentalmassen nach dem vorangehenden Anspruch, wobei als Komponente (A) Polysäuren zusammen mit mindestens einem Salz mindestens einer Polysäure eingesetzt werden, wobei die verwendeten Polysäuren mindestens einen Verzweigungspunkt in der Polymerstruktur besitzen sowie wasserlöslich und unvernetzt sind und bezüglich der Wiederholungseinheiten zu mindestens 80 Mol-% aus Acrylsäuresegmenten bestehen.

6. Dentalmassen nach Anspruch 1, umfassend:

(A) 1 bis 60 Gew.-% Polysäuren, wobei die verwendeten Polysäuren mindestens einen Verzweigungspunkt in der Polymerstruktur besitzen sowie wasserlöslich und unvernetzt sind und bezüglich der Wiederholungseinheiten zu mindestens 80 Mol-% aus Acrylsäuresegmenten bestehen, wobei unter Verzweigungspunkt eine Stelle im Polymerrückgrat zu verstehen ist, von der kovalent angebunden mindestens zwei Polymeräste ausgehen, wobei diese mindestens drei Wiederholungseinheiten enthalten;
(C) 0 bis 20 Gew.-% Zusatz- und Hilfsstoffe;
(D) 10 bis 40 Gew.-% Wasser;
(E) 30 bis 80 Gew.-% Zinkoxid.

7. Dentalmassen nach dem vorangehenden Anspruch, wobei als Komponente (A) Polysäuren zusammen mit mindestens einem Salz mindestens einer Polysäure eingesetzt werden, wobei die verwendeten Polysäuren mindestens einen Veizweigungspunkt in der Polymerstruktur besitzen sowie wasserlöslich und unvernetzt sind und bezüglich der Wiederholungseinheiten zu mindestens 80 Mol-% aus Acrylsäuresegmenten bestehen.

8. Dentalmassen nach Anspruch 1, umfassend:

(A) 1 bis 76 Gew.-% Polysäuren, wobei die verwendeten Polysäuren mindestens einen Verzweigungspunkt in der Polymerstruktur besitzen sowie wasserlöslich und unvernetzt sind und bezüglich der Wiederholungseinheiten zu mindestens 80 Mol-% aus Acrylsäuresegmenten bestehen, wobei unter Verzweigungspunkt eine Stelle im Polymerrückgrat zu verstehen ist, von der kovalent angebunden mindestens zwei Polymeräste ausgehen, wobei diese mindestens drei Wiederholungseinheiten enthalten;
(D) 5 bis 40 Gew.-% Wasser;
(F) 8,9 bis 70 Gew.-% eines oder mehrerer radikalisch polymerisierbarer Monomere;
(G) 10 bis 90 Gew.-% Füllstoffe;
(H) 0,1 bis 5 Gew,-% Initiatoren und gegebenenfalls Aktivatoren;
(1) 0 bis 30 Gew.-% Additive, gegebenenfalls Pigmente, Thixotropiehilfsmittel, Weichmacher.

9. Dentalmassen nacht dem vorangehenden Anspruch, wobei als Komponente (A) Polysäuren zusammen mit mindestens einem Salz mindestens einer Polysäure eingesetzt werden, wobei die verwendeten Polysäuren mindestens einen Verzweigungspunkt in der Polymerstruktur besitzen sowie wasserlöslich und unvernetzt sind und bezüglich der Wiederholungseinheiten zu mindestens 80. Mol-% aus Acrylsäuresegmenten bestehen.

10. Behältnis, umfassend mindestens eine Komponente einer Dentalmasse nach mindestens einem der vorangehenden Ansprüche.

11. Verwendung von wasserlöslichen und unvernetzten Polysäuren mit mindestens einem Verzweigungspunkt in der Polymerstruktur die bezüglich der Wiederholungseinheiten zu mindestens 80 Mol-% aus Acrylsäuresegmenten bestehen, wobei unter Verzweigungspunkt eine Stelle im Polymerrückgrat zu verstehen ist, von der kovalent angebunden mindestens zwei Polymeräste ausgehen, wobei diese mindestens drei Wiederholungseinheiten enthalten zur Herstellung von Dentalmassen mit verringerter Neigung zur Gelierung.

**Claims**

1. Dental materials comprising at least one polyacid or at least one polyacid together with at least one salt of at least one polyacid, the polyacids employed having at least one branching point in the polymer structure, being water-soluble and noncrosslinked and, with regard to the repeat units, comprising at least 80 mol% acrylic acid segments, the term "branching point" being understood as meaning a position in the polymer backbone from which, covalently bonded, at least two polymer residues start out, these comprising at least three repeat units.

2. Dental materials according to Claim 1, the polyacids used representing star polymers or comb polymers.

3. Dental materials according to either of the preceding claims, the polymeric acids representing copolymers in which the proportion of the comonomers amounts to 0.1 to 20 mol%.

4. Dental materials according to Claim 1, including:

   (A) 1 to 60% by weight of polyacids, in which the polyacids used have at least one branching point in the polymer structure, are water-soluble and noncrosslinked and, with regard to the repeat units, comprise at least 80 mol% acrylic acid segments, the term "branching point" being understood as meaning a position in the polymer backbone from which, covalently bonded, at least two polymer residues start out, these comprising at least three repeat units;
   (B) 35 to 80% by weight of fillers;
   (C) 0 to 20% by weight of additives and auxiliaries;
   (D) 5 to 40% by weight of water.

5. Dental materials according to the preceding claim, polyacids, together with at least one salt of at least one polyacid, being used as component (A), in which the polyacids used have at least one branching point in the polymer structure, are water-soluble and noncrosslinked and, with regard to the repeat units, comprise at least 80 mol% acrylic acid segments.

6. Dental materials according to Claim 1, including:

   (A) 1 to 60% by weight of polyacids, in which the polyacids used have at least one branching point in the polymer structure, are water-soluble and noncrosslinked and, with regard to the repeat units, comprise at least 80 mol% acrylic acid segments, the term "branching point" being understood as meaning a position in the polymer backbone from which, covalently bonded, at least two polymer residues start out, these comprising at least three repeat units;
   (C) 0 to 20% by weight of additives and auxiliaries;
   (D) 10 to 40% by weight of water;
   (E) 30 to 80% by weight of zinc oxide.

7. Dental materials according to the preceding claim, polyacids, together with at least one salt of at least one polyacid, being used as component (A), in which the polyacids used have at least one branching point in the polymer structure, are water-soluble and noncrosslinked and, with regard to the repeat units, comprise at least 80 mol% acrylic acid segments.

8. Dental materials according to Claim 1, including:

   (A) 1 to 76% by weight of polyacids, in which the polyacids used have at least one branching point in the polymer structure, are water-soluble and noncrosslinked and, with regard to the repeat units, comprise at least 80 mol% acrylic acid segments, the term "branching point" being understood as meaning a position in the polymer backbone from which, covalently bonded, at least two polymer residues start out, these comprising at least three repeat units;
   (D) 5 to 40% by weight of water;
   (F) 8.9 to 70% by weight of one or more radically polymerizable monomers;
   (G) 10 to 90% by weight of fillers;
   (H) 0.1 to 5% by weight of initiators and optionally activators;
   (I) 0 to 30% by weight of additives, optionally pigments, thixotropic auxiliaries, plasticizers.

**9.** Dental materials according to the preceding claim, polyacids, together with at least one salt of at least one polyacid, being used as component (A), in which the polyacids used have at least one branching point in the polymer structure, are water-soluble and noncrosslinked and, with regard to the repeat units, comprise at least 80 mol% acrylic acid segments.

**10.** Container including at least one component of a dental material according to at least one of the preceding claims.

**11.** Use of water-soluble and noncrosslinked polyacids with at least one branching point in the polymer structure, which with regard to the repeat units comprise at least 80 mol% acrylic acid segments, the term "branching point" being understood as meaning a position in the polymer backbone from which, covalently bonded, at least two polymer residues start out, these comprising at least three repeat units, in the preparation of dental materials with a reduced tendency to gel.

**Revendications**

**1.** Masses dentaires, contenant au moins un polyacide ou au moins un polyacide avec au moins un sel d'au moins un polyacide, les polyacides utilisés présentant au moins un point de ramification dans la structure polymère et étant solubles dans l'eau et non réticulés et étant constitués, en ce qui concerne les unités répétitives, par au moins 80% en mole de segments d'acide acrylique, en entendant par point de ramification un site dans l'épine dorsale du polymère dont partent, en étant liées par covalence, au moins deux branches polymères, celles-ci contenant au moins trois unités répétitives.

**2.** Masses dentaires selon la revendication 1, les polyacides utilisés étant des polymères en étoile ou en peigne.

**3.** Masses dentaires selon l'une quelconque des revendications précédentes, les acides polymères étant des copolymères, la proportion de comonomères étant de 0,1 à 20% en mole.

**4.** Masses dentaires selon la revendication 1, comprenant :

(A) 1 à 60% en poids de polyacides, les polyacides utilisés présentant au moins un point de ramification dans la structure polymère et étant solubles dans l'eau et non réticulés et étant constitués, en ce qui concerne les unités répétitives, par au moins 80% en mole de segments d'acide acrylique, en entendant par point de ramification un site dans l'épine dorsale du polymère dont partent, en étant liées par covalence, au moins deux branches polymères, celles-ci contenant au moins trois unités répétitives.
(B) 35 à 80% en poids de charges ;
(C) 0 à 20% en poids d'additifs et d'adjuvants ;
(D) 5 à 40% en poids d'eau.

**5.** Masses dentaires selon la revendication précédente, en utilisant comme composant (A) des polyacides avec au moins un sel d'au moins un polyacide, les polyacides utilisés présentant au moins un point de ramification dans la structure polymère et étant solubles dans l'eau et non réticulés et étant constitués, en ce qui concerne les unités répétitives, par au moins 80% en mole de segments d'acide acrylique.

**6.** Masses dentaires selon la revendication 1, comprenant :

(A) 1 à 60% en poids de polyacides, les polyacides utilisés présentant au moins un point de ramification dans la structure polymère et étant solubles dans l'eau et non réticulés et étant constitués, en ce qui concerne les unités répétitives, par au moins 80% en mole de segments d'acide acrylique, en entendant par point de ramification un site dans l'épine dorsale du polymère dont partent, en étant liées par covalence, au moins deux branches polymères, celles-ci contenant au moins trois unités répétitives.
(C) 0 à 20% en poids d'additifs et d'adjuvants ;
(D) 10 à 40% en poids d'eau ;
(E) 30 à 80% en poids d'oxyde de zinc.

**7.** Masses dentaires selon la revendication précédente, en utilisant comme composant (A) des polyacides avec au moins un sel d'au moins un polyacide, les polyacides utilisés présentant au moins un point de ramification dans la structure polymère et étant solubles dans l'eau et non réticulés et étant constitués, en ce qui concerne les unités répétitives, par au moins 80% en mole de segments d'acide acrylique.

8. Masses dentaires selon la revendication 1, comprenant :

(A) 1 à 76% en poids de polyacides, les polyacides utilisés présentant au moins un point de ramification dans la structure polymère et étant solubles dans l'eau et non réticulés et étant constitués, en ce qui concerne les unités répétitives, par au moins 80% en mole de segments d'acide acrylique, en entendant par point de ramification un site dans l'épine dorsale du polymère dont partent, en étant liées par covalence, au moins deux branches polymères, celles-ci contenant au moins trois unités répétitives.
(D) 5 à 40% en poids d'eau ;
(F) 8,9 à 70% en poids d'un ou de plusieurs monomères polymérisables par voie radicalaire ;
(G) 10 à 90% en poids de charges ;
(H) 0,1 à 5% en poids d'initiateur et le cas échéant d'activateurs ;
(I) 0 à 30% en poids d'additifs, le cas échéant des pigments, des adjuvants thixotropiques et des plastifiants.

9. Masses dentaires selon la revendication précédente, en utilisant comme composant (A) des polyacides avec au moins un sel d'au moins un polyacide, les polyacides utilisés présentant au moins un point de ramification dans la structure polymère et étant solubles dans l'eau et non réticulés et étant constitués, en ce qui concerne les unités répétitives, par au moins 80% en mole de segments d'acide acrylique.

10. Conteneur, comprenant au moins un composant d'une masse dentaire selon l'une quelconque des revendications précédentes.

11. Utilisation de polyacides solubles dans l'eau et non réticulés présentant au moins un point de ramification dans la structure polymère qui sont constitués, en ce qui concerne les unités répétitives, par au moins 80% en mole de segments d'acide acrylique, en entendant par point de ramification un site dans l'épine dorsale du polymère dont partent, en étant liées par covalence, au moins deux branches polymères, celles-ci contenant au moins trois unités répétitives, pour la préparation de masses dentaires avec une tendance diminuée à la gélification.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2061513 A1
- EP 0024056 A1
- US 4174334 A
- US 5141560 A
- EP 0219058 A
- JP 4221303 A
- JP 4221304 A
- DE 2061513 A
- DE 2065824 A
- DE 2929121 A
- DE 2319715 A
- US 3066112 A
- DE 2816823 C

- DE 4445266 A
- EP 0073413 A
- EP 0007508 A
- EP 0047902 A
- EP 0057474 A
- EP 0184095 A
- US 3541068 A
- DE 2658530 A
- DE 1495520 C
- EP 0059451 A
- DE 19928238 A
- EP 0374824 A


**In der Beschreibung aufgeführte Nicht-Patentliteratur**

- **D. C. SMITH.** *Br. Dent. J.,* 1968, vol. 125, 381-384
- **WILSON ; KENT.** *ASPA I,* 1969
- **AUS S. CRISP ; B. E. KENT ; B. G. LEWIS ; A. J. FERNER ; A. D. WILSON.** *J. Dent. Res.,* 1980, vol. 59, 1055-1063
- **K. MATYJASZEWSKI et al.** Arm-first'' und ''Core-first. *Macromolecules,* 1999, vol. 32, 6526
- **A. AKAR ; N. ÖZ.** *Angew. Makromol. Chem.,* 1999, vol. 273, 12-14
- **K. A. DAVIS et al.** *Polymer Prepr.,* 1999, vol. 2, 430

- **P. BANERJEE ; A. M. MAYES.** *Macromolecules,* 1998, vol. 31, 7966
- **C. PUGH et al.** *Polymer Prepr.,* 1999, vol. 2, 108
- **D. M. HADDLETON et al.** *New J. Chem.,* 1999, vol. 23, 477
- **HEISE et al.** *Polymer Prepr.,* 1999, vol. 1, 452
- **A. HEISE et al.** *Macromolecules,* 1999, vol. 32, 231
- **S. ANGOT et al.** *Macromolecules,* 1998, vol. 31, 7218